# FASCICULE DE BREVET EUROPEEN

(11) **EP 0 779 103 B1**
(45) Date de publication et mention de la délivrance du brevet: **24.04.2002**
(21) Numéro de dépôt: 96420352.5
(22) Date de dépôt: 09.12.1996
(51) Int. Cl.: B01L 3/00, G01N 33/80

(54) **Dispositif de contrôle visuel d'un liquide par mélange avec un liquide réactif**
Vorrichtung zur visuellen Untersuchung einer Flüssigkeit durch Vermengen mit einem flüssigen Reagens
Device for visually assaying a liquid by mixing with a liquid reagent

(30) Priorité: 13.12.1995 FR 9514945
(43) Date de publication de la demande: 18.06.1997
(73) Titulaire: Pharmabio S.à.r.l., 78100 St Germain en Laye (FR)
(72) Inventeur: Bonnier, François, 69130 Ecully (FR); Depaulis, Robert, 78112 Fourqueux (FR)
(74) Mandataire: Schmitt, John

(56) Documents cités:
- EP-A- 0 054 087
- EP-A- 0 396 115
- EP-A- 0 678 745
- WO-A-95/27196
- US-A- 5 342 581

## Description

L'invention concerne un dispositif de contrôle visuel d'un liquide par mélange avec un liquide réactif. Ce dispositif s'applique au test de contrôle prétransfusionnel du groupe sanguin, par exemple, mais non exclusivement.

Lors d'une intervention chirurgicale ou d'un accident, lorsqu'un patient a besoin d'une transfusion sanguine il est nécessaire de contrôler la compatibilité du sang de ce dernier avec celui du donneur; cette opération de contrôle, qui se fait au lit du malade, est appelée ci-après "contrôle" prétransfusionnel". La poche qui est livrée comporte entre autres choses un tuyau de petit diamètre, qu'on appellera ci-après "nouille témoin" contenant le même sang que celui qui est dans la poche et qui est marqué à intervalles réguliers d'un numéro d'identification identique à celui imprimé sur la poche; les infirmières qui ont fait le prélèvement écrasent et ferment le tube par soudure entre des électrodes chaudes à intervalles réguliers dans les zones séparant celles portant le numéro d'identification; elles constituent ainsi des petits réservoirs de sang portant un numéro d'identification. Au moment où l'on va utiliser la poche de sang, on prélève un de ces petits réservoirs en le séparant de la poche en coupant le tube à l'endroit où il est soudé. Une manière de réaliser ce contrôle consiste à prélever une nouille témoin qui permettra de faire le contrôle sans pollution de la poche de sang; le principe du test consiste à mélanger le sang avec un liquide appelé sérum-test, sur une plaque opaline ou une assiette ou tout autre support de même nature, afin d'identifier soit une réaction immédiate d'agglutination, soit une absence d'agglutination. Pour réaliser ce test on utilise deux sérums-test différents appelés "Anti-A" et "Anti-B"; on dépose sur le support choisi deux fois une goutte de chacun des deux sérums-test; on mélange une goutte du sang du donneur avec une des gouttes de chacun des sérums-test, et on fait de même avec le sang du receveur; les sangs sont compatibles si les réactions de chacun des sangs avec chacun des sérums-tests sont identiques. La mise en oeuvre de ce test présente un risque de contamination d'une goutte de sérum-test par l'autre sérum-test et un risque de confusion dans la lecture des résultats obtenus parce que les gouttes de sérums-test ne sont pas clairement identifiées; pour essayer de pallier le risque de confusion, il est proposé notamment une carte vernie imprimée et comportant notamment des zones délimitées par des cercles ou des carrés constituant une ligne de test par échantillon de sang à contrôler; une ligne de test comprend à gauche un carré dans lequel on dépose une goutte du sang à comparer et à droite deux ronds dans lesquels on dépose, dans le rond de gauche une goutte d'un "sérum-test Anti A" et dans le rond de droite une goutte d'un "sérum-test Anti B"; ces gouttes sont prélevées dans des flacons. Il y a de deux à quatre lignes de test; la première ligne de test concerne le sang du receveur et les lignes de test suivantes concernent les poches de sang destinées à être transfusées; en ce qui concerne le sang transfusé la goutte de sang est extraite de la nouille témoin prélevée sur la poche, et qu'on a ouvert en en coupant au moins une extrémité avec une paire de ciseaux. La carte comprend, en outre, notamment des informations concernant la personne qui a fait le contrôle prétransfusionnel et elles est archivée dans le dossier du malade. Cette carte présente l'inconvénient de ne pas éliminer le risque de pollution d'un sérum-test par l'autre, ni le risque d'inversion des sérums-test; notamment le sérum-test des bouteilles peut être dégradé s'il est resté trop longtemps hors d'un réfrigérateur ou bien avoir été pollué au moment du prélèvement de la goutte dans une opération précédente. Une autre carte comporte des sérums-test déshydratés placés préalablement dans les cases pour éliminer le risque d'inversion, mais le risque de pollution demeure au moment de leur réhydratation.

Cette façon de procéder pour réaliser le contrôle prétransfusionnel présente l'inconvénient de ne pas être facile à mette en oeuvre au chevet d'un patient parce qu'il faut manipuler avec soin de nombreux accessoires dans une atmosphère où l'urgence n'est pas propice à la fiabilité du contrôle prétransfusionnel.

L'objet de l'invention est de proposer un procédé opératoire ainsi qu'un dispositif ambulatoire permettant de fiabiliser les contrôles prétransfusionnels quelles que soient les conditions de mises en oeuvres et de permettre en outre un contrôle à postériori des résultats du contrôle prétransfusionnel effectué.

Par le document WO-A-9527196, un dispositif est connu qui comprend plusieurs alvéoles qui peuvent contenir un liquide différent et qui sont refermables par un dispositif indépendant de bouchage manuel comportant des bouchons verrouillés sur les alvéoles au moyen d'une liaison mécanique.

Par le brevet EP-A-0054087, on connait aussi un dispositif comprenant des flasques munis de dents ayant des alvéoles percants reliés par une liaison mécanique.

Ces dispositifs ne répondent pas aux préoccupations de pollution, car ils ne permettent pas d'éviter les inversions de bouchage des alévoles.

Au contraire, le dispositif selon l'invention évite la pollution par le bouchage en associant mécaniquement chaque bouchon à chaque alvéole sans que des inversions de bouchage soient possibles.

Sur les dessins :

La figure 1 représente en vue perspective un dispositif de test ambulatoire selon l'invention comprenant deux modules de contrôle prétransfusionnels selon l'invention dont l'un a été découpé pour en montrer la structure.

La figure 2 représente en vue perspective un dispositif de test ambulatoire selon une version préférée de l'invention comprenant quatre modules de contrôles prétransfusionnels en position ouverte ou fermée dont l'un des modules est partiellement découpé pour en montrer la structure.

La figure 3 représente une coupe du dispositif selon la figure 1 en position refermée avec la nouille témoin dans son logement de stockage.

La figure 4 représente une vue en perspective du dispositif de la figure 2 lorsqu'il est replié sur lui-même pour former une pochette.

La figure 5 représente en vue perspective un alvéole suivant une version préférée de l'invention.

La figure 6 représente une coupe verticale d'un alvéole formé d'un conteneur indépendant fermé et clipsé sur un support. Le dispositif de rebouchage n'est pas représenté.

La figure 7 représente une vue perspective partiellement éclatée de deux alvéoles communiquant par des canaux avec des logements contenant chacun un conteneur de liquide réactif. Le dispositif de bouchage et rebouchage des alvéoles n'est pas représenté.

Le procédé consiste à réaliser le test par apport et mélange d'un liquide à contrôler dans des cavités d'un dispositif contenant chacune un réactif liquide qui y a été introduit au moment de son conditionnement en usine et dont le résultat est observé visuellement directement dans chaque cavité au niveau de caractéristiques physiques telles que la consistance et/ou la couleur sans que cette liste soit limitative; la cavité est ensuite refermée hermétiquement pour conserver le résultat obtenu par le mélange ainsi réalisé. Le liquide réactif est introduit en quantité nécessaire et suffisante dans un alvéole, dans lequel il est enfermé et conservé; il y a autant d'alvéoles qu'il y a de liquides réactifs différents nécessaires pour réaliser le test et l'ensemble de ces alvéoles constituent un module de test; il faut un module de test par liquide à tester.

Ce procédé peut être appliqué à la réalisation du test prétransfusionnel dans lequel le liquide à tester est du sang et les liquides réactifs du sérum-test Anti-A et du sérum-test Anti-B; on mélange le sang du receveur dans chacune des cavités d'un module de deux alvéoles qui ont été préalablement ouverts et puis on observe le résultat qui se traduit soit par une agglutination soit par l'absence d'agglutination et on referme les alvéoles; ensuite on mélange le sang du donneur provenant d'une nouille témoin prélevée sur la poche; dans chacune des cavités d'un autre module de deux alvéoles qui ont été préalablement ouverts et puis qui seront ensuite refermés après observation visuelle du résultat. Le risque de pollution d'un sérum-test par l'autre est éliminé et le risque de pollution d'un sang par l'autre est éliminé. Dans ce qui suit, à titre d'exemple non-limitatif du domaine de l'invention tel que décrit précédemment, on va décrire un dispositif permettant de réaliser un test prétransfusionnel.

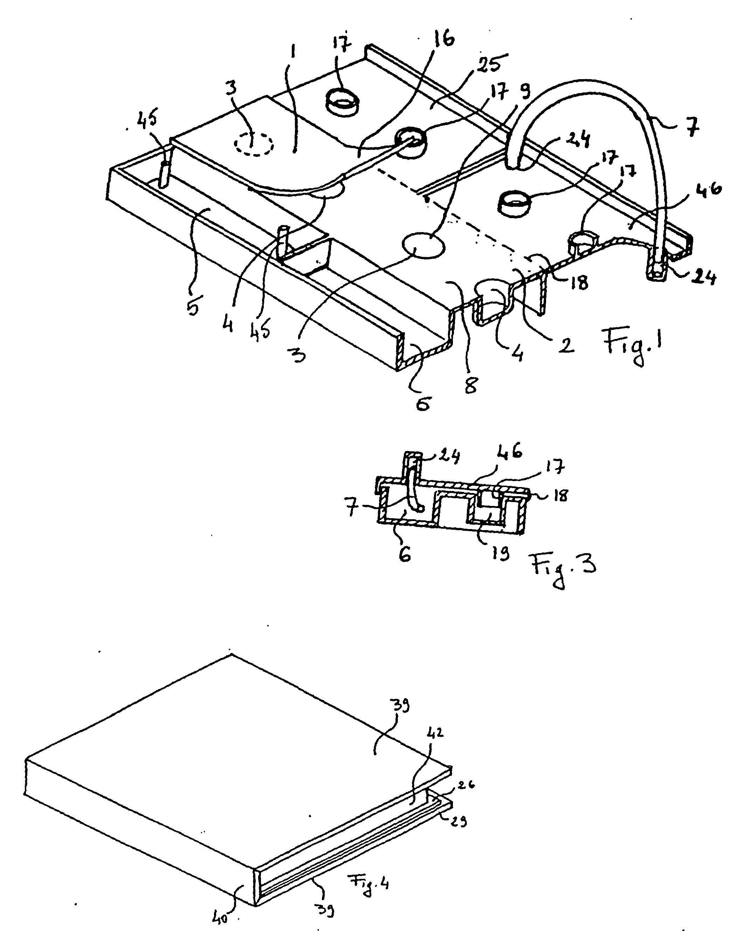

Le dispositif de test contient au moins deux modules 1 et 2 (fig.1) d'au moins deux alvéoles 3 et 4 fermés contenant respectivement du sérum-test Anti-A et du sérum-test Anti-B; chaque module 1 et 2 d'alvéoles 3 et 4 comporte en outre un dispositif de stockage 5 et 6, après utilisation, de la nouille témoin 7 correspondante ayant servi au test dans le module d'alvéoles 3 et 4 correspondant; ce dispositif de stockage est conçu pour empêcher que la nouille témoin soit perdue par inadvertance et ne puisse être récupérée que par un acte volontaire. Le contrôle prétransfusionnel consiste à ouvrir un premier module 1 de deux alvéoles 3 et 4 pour y introduire une goutte de sang du receveur; ensuite. d'ouvrir un deuxième module 2 d'alvéoles 3 et 4 pour y introduire le sang provenant d'une nouille témoin 7, prélevée sur la poche de sang à transfuser correspondante; ensuite, on compare la réaction des sangs avec les sérums-test afin de vérifier si elles sont bien identiques; et on recommence avec d'autres modules de deux alévoles non utilisés si on a besoin de poches de sang supplémentaires. Dans tout ce qui suit on va considérer que le module comporte deux alvéoles, mais si un nombre d'alvéoles plus important est nécessaire, cela fait toujours partie du domaine de l'invention.

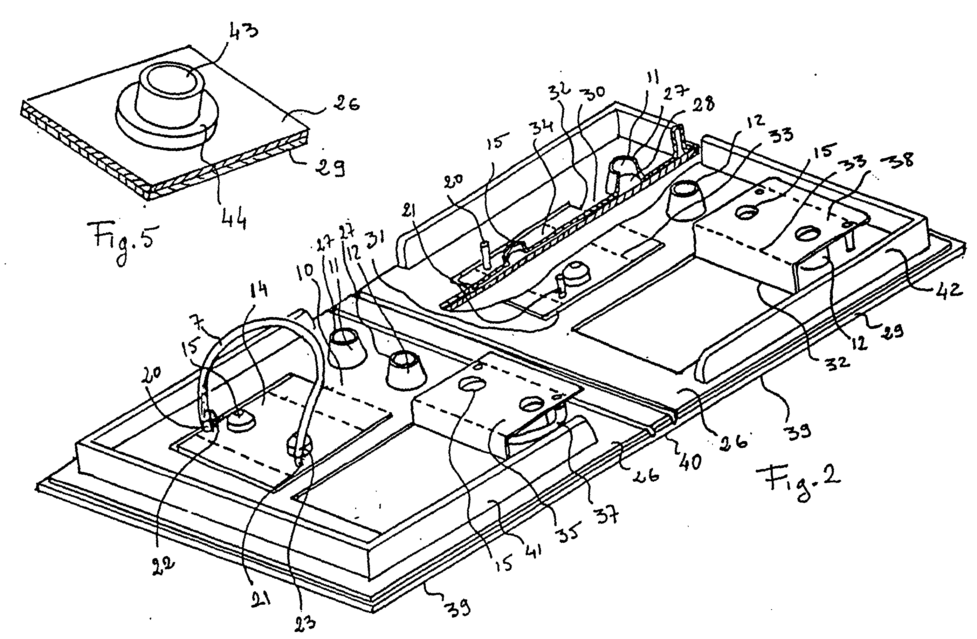

Les alvéoles 3 et 4 sont de préférence réalisés dans des matières thermoplastiques injectées (fig.1) ou thermoformées (fig.2) et se présentent généralement disposés par groupe de deux sur une plaque plane 8 (fig.1) l'ouverture 9 des alvéoles 3 et 4 peut se situer aussi bien au niveau de la plaque 8 avec les alvéoles 3 et 4 encastrés dans cette dernière ou bien au contraire les alvéoles 11 et 12 (fig.2) du module 10 forment une verrue sur la plaque principale 26; les choix se font en tenant compte des solutions techniques retenues pour les diverses fonctions de bouchage des alvéoles 3 et 4 ou 11 et 12 et de stockage avec dispositif d'inviolabilité des nouilles de sang 7 ayant servi au test prétransfusionnel.

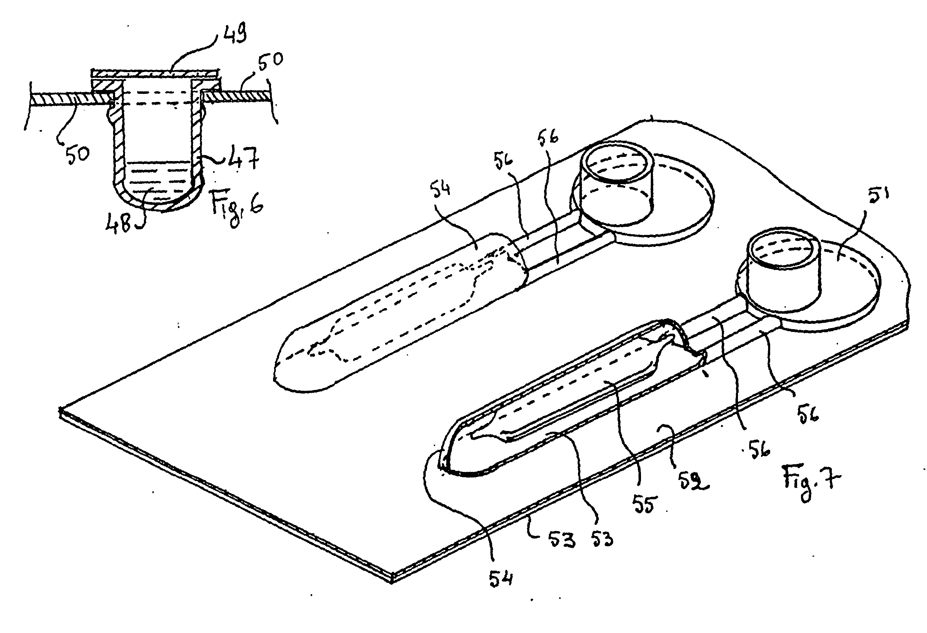

Dans le but de permettre un éventuel contrôle à postériori du test prétransfusionnel, dans une variante de l'invention, les alvéoles 3 et 4 sont refermés après introduction du sang, pour garder la trace du résultat du contrôle prétransfusionnel effectué; dans une version préférée de l'invention, c'est le même dispositif de bouchage 14 (fig.5) manuel qui sert à obturer les alvéoles 11 et 12 après introduction des sérums-test et après réalisation du contrôle prétransfusionnel; il est conçu de manière qu'il y ait un dispositif de bouchage 14 associé à chaque groupe d'alvéoles 11 et 12 sans qu'il y ait possibilité d'interchanger les bouchons 15 pour constituer un module 10. Dans une variante de l'invention, on utilise un dispositif de bouchage, éventuellement inviolable et non réutilisable, facile à poser industriellement après introduction des sérums-test dans les groupes d'alvéoles, associé à un autre système de bouchage manuel après le contrôle prétransfusionnel; à titre d'exemple non limitatif, les alvéoles 3 et 4 (fig.1) peuvent être obturés après remplissage avec le sérum-test par une pellicule 16 (fig.1) soudée et qui peut être enlevée par pelage; des bouchons 17 fixés à chaque groupe d'alvéoles 3 et 4 correspondant par une liaison mécanique souple 18 permettent de refermer les alvéoles 19 (fig.3) manuellement après exécution du test.

Dans le même but de permettre un éventuel contrôle à postériori, chaque groupe de deux alvéoles 2 et 4 (fig.1) sauf éventuellement celui qui est destiné au sang du receveur, comporte un dispositif de stockage 6 de la nouille témoin 7 contenant du sang ayant servi au test muni d'un dispositif d'inviolabilité; il est réalisé de manière que la nouille témoin 7 ne puisse être perdue par inadvertance ou attribuée à un autre groupe de deux alvéoles 3 et 4; à titre d'exemple non limitatif, dans une version préférée de l'invention, une manière de stocker la nouille témoin 7 (fig.2) consiste à associer à chaque groupe de deux alvéoles 11 et 12 des supports d'extrémités 20 et 21 de la nouille témoin 7 constitués, par exemple, de deux petites tiges cylindriques et/ou coniques, fixées au voisinage immédiat des alvéoles 11 et 12, dont le diamètre maximum est de préférence légèrement supérieur au diamètre interne des nouilles témoins 7 utilisées; on ouvre une extrémité 22 de la nouille témoin 7 qu'on fixe sur le support d'extrémité 20 on coupe l'autre extrémité 23 pour en extraire la quantité de sang nécessaire au contrôle à effectuer, par exemple, en pressant la nouille témoin 7 entre les doigts; ensuite on enfile l'autre extrémité 23 de la nouille témoin 7 sur le support d'extrémité 21. Les petites tiges, constituant les supports d'extrémités 20 et 21 des nouilles témoins 7, peuvent être remplacées par des trous cylindriques aveugles 24 (fig.1) dont le diamètre est légèrement inférieur au diamètre extérieur de la nouille témoin 7 avec éventuellement une entrée évasée pour permettre d'y introduire l'extrémité de la nouille témoin 7. Les supports d'extrémités 45 ou 24 (fig.1) peuvent être placés soit dans la cavité de stockage 5 de la nouille témoin 7 soit sur le dispositif de bouchage manuel 46 des alvéoles 3 et 4; le dispositif de bouchage 46 (fig.3) sert alors aussi de couvercle de fermeture de la cavité de stockage 6.

Une fois la nouille témoin fixée à ses extrémités après usage, dans une variante de l'invention la nouille témoin est enfermée dans une cavité dont l'accès est protégé par un dispositif inviolable de manière qu'elle ne puisse être enlevée que volontairement. A titre d'exemple non limitatif, dans une version préférée de l'invention, le dispositif de bouchage 46 manuel (fig.3), supportant les bouchons 17 des alvéoles, après réalisation du test, qui est associé à un groupe d'alvéoles 19 par une liaison mécanique souple 18, peut constituer un couvercle venant enfermer la nouille témoin 7 en place sur ses supports d'extrémités 24 dans la cavité de stockage 6; en refermant les alvéoles 19 avec les bouchons 17, on emprisonne en même temps la nouille témoin 7 dans la cavité de stockage 6 qui peut être rendue inviolable par un dispositif d'inviolabilité associé au dispositif de bouchage 46, ce dispositif d'inviolabilité n'est pas représenté sur la figure 3.

Dans une version préférée de l'invention, les alvéoles 11 et 12 (fig.2) sont réalisés par thermoformage d'une plaque principale 26 de matière thermoplastique de manière que chaque alvéole 11 et 12 forme un bulbe en excroissance par rapport au plan de la plaque principale 26; la partie supérieure des bulbes est découpée au niveau d'une zone ayant une forme cylindrique pour former un orifice 27; le fond 28 des alvéoles 11 et 12 est formé par une plaque associée 29 de matière thermoplastique rapportée et soudée à la plaque principale 26 notamment tout autour des bulbes formant les alvéoles 11 et 12. Le dispositif selon l'invention est constitué d'un assemblage de modules de test identiques identifiés par les repères 10,30,35,38; ces modules sont présentés (fig.2) dans des configurations différentes en position ouverte ou fermée; le module 30 est en position initiale avant l'introduction des sérums-test; le module 38 est fermé tel qu'il se présente avant le test prétransfusionnel; le module 10 est représenté en fin de test prétransfusionnel avec la nouille témoin fixée à ses extrémités; le module 35 est représenté en position de classement avec la nouille témoin dans sa zone de stockage; chacun de ces modules de test tel que le module de test 30 étant constitué d'un ensemble comprenant deux alvéoles 11 et 12 et deux bouchons 15 ces derniers étant formés par thermoformage dans la même plaque principale 26 d'une partie cylindrique, de préférence de révolution, continuée vers le haut d'une partie tronconique, de préférence de révolution, dont la partie supérieure est fermée et de préférence plane; la plaque principale 26 est découpée autour de ces bouchons 15, suivant un tracé 31 sensiblement rectangulaire, sur trois côtés pour constituer le dispositif de bouchage 34 supportant les deux bouchons 15; le dernier côté, situé entre les bouchons 15 et les alvéoles 11 et 12, est articulé à l'aide d'au moins deux lignes 32 et 33 de prédécoupe parallèles au plan formé par les axes de révolution des deux bouchons 15; c'est en pliant la plaque principale 26 au niveau des deux prédécoupes 32 et 33 qu'on amène les bouchons 15 au-dessus de l'orifice 27 des alvéoles 11 et 12 pour les fermer en introduisant la partie cylindrique des bouchons 15 dans les orifices 27, comme le montrent les modules de test 35 et 38 qui ont leur dispositif de bouchage refermé. Le dispositif de bouchage 34 peut comporter deux petites excroissances cylindriques et/ou coniques formées aussi par thermoformage et constituant les supports d'extrémité 20 et 21 de nouilles témoins 7 précédemment décrites pour y fixer les extrémités de la nouille témoin 7; le dispositif de bouchage 14 des alvéoles du module 10 est suffisament grand pour recouvrir la quasi totalité de la nouille témoin 7 lorsque ses extrémités 22 et 23 sont fixées sur les supports d'extrémité 20 et 21 correspondants et que les bouchons 15 sont en place sur les orifices des alvéoles correspondants comme le montre le module 35 dont le dispositif de bouchage 36 est refermé sur les alvéoles en emprisonnant la nouille témoin 37; les supports d'extrémité sont suffisamment long pour coincer la nouille contre la paroi de la plaque principale 26 de manière que la nouille témoin 37 soit coincée et ne puisse être enlevée sans dégrader le support d'extrémité correspondant.

Les alvéoles sont fermés à leur base par une plaque associée 29 de matière thermoplastique plane qui est soudée sur la partie plane de la plaque principale 26 au moins autour des alvéoles tout en laissant libre de toute soudure les dispositifs de bouchage des alvéoles.

Les modules de test sont assemblés par deux, trois ou quatre modules sur une même plaque principale 26 (fig.2) les fonds des alvéoles sont formés par une plaque associée 29 ayant sensiblement la même surface que la plaque principale 26 l'ensemble ainsi constitué pouvant être replié sur lui-même en portefeuille de manière qu'il puisse y avoir jusqu'à deux modules de chaque côté 39 (fig.4) de la pochette ainsi formée, séparés par une zone charnière médiane 40. Les bords de la plaque principale 26 (fig.2) entourant chaque côté 39 de la pochette comportent des excroissances 41 et 42 qui s'emboîtent l'une dans l'autre lorsqu'on ferme la pochette (fig.4) et qui se verrouillent suffisamment pour maintenir la pochette fermée.

Dans une version préférée de l'invention, la plaque principale 26 (fig.5) est en matière plastique transparente thermoformable et de même nature que la plaque associée 29; les alvéoles 43 présentent à leur base un renflement annulaire 44 créant ainsi une faible épaisseur de liquide permettant de bien voir à travers la paroi de la plaque principale 26 la couleur du liquide contenu dans l'alvéole 43 examiné, après introduction de la goutte de sang; les bouchons 15 (fig.2) qui referment les alvéoles 11 et 12, après introduction de la goutte de sang, ont une hauteur qui est suffisamment importante pour, lorsqu'ils sont en place dans leur alvéole 11 ou 12 respective, que leur sommet s'approche du fond de l'alvéole et occupe un volume suffisant pour écraser et disperser la goutte de sang, la mêler au sérum-test et en refouler une partie vers le renflement 44 (fig.5) afin de créer un film de sang réagissant rapidement avec le sérum-test du fait de l'importance de la surface de contact des deux liquides en présence. Dans le but d'améliorer la vitesse de réaction et la lecture du résultat, la face de la plaque associée 29 qui constitue le fond de l'alvéole 11 ou 12 présente une surface légèrement rugueuse. Les plaques 26 et 29 peuvent être, par exemple, sans que cet exemple soit limitatif, en polychlorure de vinyle rigide et être soudées entre elles, par exemple, sans que cet exemple soit limitatif, par la technique de soudure par haute fréquence.

Les matériaux qui constituent les parois des alvéoles doivent permettre une conservation des liquides réactifs suffisamment longue; en effet, avec le temps, les liquides réactifs peuvent être oxydés par contact avec l'air résiduel ou migrant à travers les parois de l'alvéole, de même que les solvants des liquides réactifs peuvent migrer à travers les parois de l'alvéole; il est donc indispensable de réaliser des alvéoles ainsi que les moyens de fermeture, avant mise en oeuvre, dans des matériaux ou des assemblages de matériaux multicouches permettant d'obtenir la durée de vie souhaitée. Dans le cas où le niveau de performance exigé nécessite l'emploi de matériaux coûteux, il est préférable de ne les utiliser que pour les alvéoles et leur moyen de fermeture avant mise en oeuvre, les autres fonctions du dispositif de test ambulatoire étant dans un matériau moins noble par rapport à la performance de conservation du liquide réactif, mais aussi mieux adpaté aux performances spécifiques à ces autres fonctions.

Comme le montre à titre d'exemple, non limitatif, la figure 6, chaque alvéole est un conteneur indépendant 47 des autres alvéoles, rempli de liquide réactif 48, fermé par un film pelable 49, fixé par clipsage sur un support de fixation 50 prévu à cet effet; le conteneur indépendant 47 est réalisé en matière plastique injectée ou thermoformée, présentant les caractéristiques nécessaires et suffisantes pour une conservation convenable du liquide réactif 48, tandis que le bouchon qui est destiné à refermer le conteneur indépendant 47 après mise en oeuvre du test est réalisé avec, par exemple, la même matière qui sert à réaliser le support de fixation 50.

Une autre manière de procéder consiste à réaliser l'alvéole comme précédemment décrit dans un matériau peu coûteux permettant de réaliser le dispositif de test ambulatoire dans des conditions de coût convenables, et à introduire dans l'alvéole, non pas le liquide réactif mais un petit conteneur contenant le liquide réactif, ce conteneur est susceptible d'être ouvert par simple pression sur ses parois au moment de la mise en oeuvre du test ambulatoire si bien que le liquide réactif ne reste que peu de temps en contact avec l'alvéole.

Dans une version préférée de l'invention représentée figure 7, l'alvéole 51 est réalisé dans une plaque principale 52 thermoformée et fermée par une plaque associée 53; on thermoforme dans la plaque principale 52, en même temps que l'alvéole 51, un logement 54 spécifique au conteneur de liquide réactif 55 qui communique par au moins un canal 56 avec l'alvéole 51, on place dans ce logement 54 le conteneur de liquide réactif 55 et on ferme l'ensemble par la plaque associée 53; le logement 54 est conçu pour permettre l'écrasement du conteneur de liquide réactif 55 par déformation manuelle de la paroi du logement 55 afin de permettre la libération du liquide réactif qui est dirigé, par les canaux 56, vers l'alvéole 51. Dans la version préférée de l'invention, le conteneur de liquide réactif 55 est une ampoule en verre qui est brisée par simple pression à travers les parois du logement 54; le liquide réactif ainsi libéré peut être dirigé vers l'alvéole 51 par gravité à travers les canaux 56, en inclinant convenablement le dispositif de test ambulatoire.

La pochette 39 peut comporter sur sa face externe des informations concernant notamment les donneurs et receveurs et les conditions d'utilisation.

## Revendications

1. Dispositif de test d'un liquide par mélange de chacun d'eux avec un ou plusieurs liquides réactifs, comprenant au moins deux modules (1,2) de deux alvéoles (3,4) contenant chacun un liquide réactif différent de manière qu'à chaque alvéole de chaque module corresponde respectivement un alvéole de chacun des autres modules contenant le même liquide réactif, les alvéoles (3,4) étant refermés, après le test, par un dispositif de bouchage (25) manuel comportant des bouchons (17), relié aux alvéoles (3,4) par un moyen mécanique, **caractérisé par le fait que** le dispositif de bouchage (25) comprend le même nombre de bouchons (17) que le module (1,2) comprend d'alvéoles, ledit dispositif de bouchage étant associé à chaque module par une liaison mécanique souple (18) formant une zone de pliage autorisant le repliement du dispositif de bouchage (25) sur les modules (1,2) évitant d'interchanger les bouchons.

2. Dispositif suivant la revendication 1, **caractérisé en ce que** la fermeture des alvéoles avant test est assuré par une pellicule (16) soudée sur le bord de l'orifice (9) des alvéoles (3,4) et susceptible d'être enlevée par pelage.

3. Dispositif suivant la revendication 1, **caractérisé en ce que** la fermeture des alvéoles (11,12) après introduction des liquides réactifs est assurée par le même dispositif de bouchage (14) manuel de refermeture des alvéoles (11,12), notamment par des bouchons (15).

4. Dispositif suivant les revendications 1 à 3, **caractérisé en ce que** l'un des alvéoles (3,4) de chaque module contient du sérum-test Anti-A et l'autre du sérum-test Anti-B, le premier module (1) étant destiné à recevoir le sang du receveur, chaque autre module (2) étant destiné à recevoir le sang d'un donneur extrait d'une nouille témoin (7).

5. Dispositif suivant la revendication 4, **caractérisé en ce qu'**une cavité refermable de stockage (6) de la nouille témoin (7) est associée à chacun des modules (2).

6. Dispositif suviant la revendication 4, **caractérisé en ce que** la nouille témoin (7) est fixée, après usage, à ses extrémités (22, 23) qui ont été découpées au cours de l'opération de test, à des supports d'extrémité (20, 21, 24) fixés soit dans une cavité de stockage (5), soit sur le dispositif de bouchage (46) servant de couvercle à la cavité de stockage (6).

7. Dispositif suivant la revendication 6, **caractérisé en ce que** le dispositif de bouchage (35), lorsqu'il est en position de fermeture des alvéoles après test, coince la nouille témoin (7) entre le support d'extrémité de forme, cylindrique et/ou conique de la nouille témoin (7), associé au couvercle et la plaque principale (26).

8. Dispositif suivant l'une quelconque des revendications précédentes, **caractérisé en ce que** les alvéoles sont réalisés par thermoformage d'une plaque thermoplastique principale (26) soudée à une plaque associée (29) pour former le fond des alvéoles (11,12), tandis que chaque module (30) comporte un dispositif de bouchage (34), supportant des bouchons (15) et des supports d'extrémités (20,21) thermoformés, découpé dans la plaque principale (26) suivant un tracé (31) comportant trois côtés et le quatrième étant articulé grâce à des lignes de prédécoupes (32, 33).

9. Dispositif .suivant la revendication 8, **caractérisé en ce que** la plaque principale (26) est transparente, la face formant le fond des alvéoles (11, 12) de la plaque associée (29) est légèrement rugueuse, les bouchons (15) pénètrent suffisamment à l'intérieur de leur alvéole (11, 12) respectif pour y écraser la goutte de sang et en refouler une partie vers un renflement (44) situé à la base des alvéoles (11, 12).

10. Dispositif suivant la revendication 1, **caractérisé en ce que** les alvéoles sont formés de conteneurs indépendants (47) clipsés sur un support de fixation (50).

11. Dispositif suivant les revendications 1 et 8, **caractérisé en ce qu'**un alvéole (51) est associé à un logement (54) communiquant avec ledit alvéole par au moins un canal (56), alvéoles et canaux étant thermoformés dans une plaque thermoplastique principale (52) et fermés par une plaque associée (53), le logement (54) contenant un çonteneur de liquide réactif (55) susceptible d'être écrasé afin de libérer le liquide réactif qu'il contient et de le diriger vers l'alvéole (51) par l'intermédiaire du canal (56).

12. Dispositif suivant les revendications 1 et 8, **caractérisé en ce qu'**il comporte de deux à quatre modules qui sont répartis suivant deux côtés (39) qui se replient sur eux-mêmes suivant une zone charnière médiane (40) pour former une pochette dont les bords sont formés d'excroissances (41, 42) thermoformées à partir de la plaque principale (26) et qui s'emboîtent pour maintenir la pochette fermée.

13. Dispositif suivant la revendication 8, **caractérisé en ce que** les plaques (26, 29) sont en polychlorure de vinyle rigide et soudées par soudure à haute fréquence.

## Claims

1. Device for testing a liquid by mixing each liquid with one or several reactive liquids, said device including at least two modules (1, 2) with two alveoles (3, 4) each containing a different reactive liquid so that each alveole of each module corresponds respectively to one alveole of each of the other modules containing the same reactive liquid, the alveoles (3,4) being closed after the test by a manual sealing device (25) comprising stoppers (17) connected to the alveoles (3, 4) by a mechanical element, **characterised by** the fact that the sealing device (25) includes the same number of stoppers (17) as the alveoles included in the module (1, 2), said sealing device being associated with each module by a flexible mechanical link (18) forming a folding zone authorising the folding of the sealing device (25) onto the modules (1, 2) avoiding interchanging the stoppers.

2. Device according to claim 1, **characterised in that** the closing of the alveoles before the test is ensured by a coating (16) welded onto the edge of the orifice (9) of the alveoles (3, 4) and able to be removed by peeling.

3. Device according to claim 1, **characterised in that** the closing of the alveoles (11, 12) following the introduction of the reactive liquids is provided by the same manual sealing device (14) for closing the alveoles (11, 12) by means in particular of the stoppers (15).

4. Device according to claims 1 to 3, **characterised in that** one of the alveoles (3, 4) of each module contains Anti-A test serum and the other Anti-B test serum, the first module (1) being used to receive the blood of the receiver, each other module (2) being used to receive the blood of a donor extracted with a reference noodle (7).

5. Device according to claim 4, **characterised in that** a closable storage cavity (6) for the reference noodle (7) is associated with each of the modules (2).

6. Device according to claim 4, **characterised in that** the reference noodle (7) is fixed after usage at its extremities (22, 23) which have been cut during the test operation to extremity supports (20, 21, 24) fixed either in a storage cavity (5) or onto the sealing device (46) used as a cover for the storage cavity (6).

7. Device according to claim 6, **characterised in that** the sealing device (35), when it is in the after test alveole closing position, jams the reference noodle (7) between the cylindrical and/or conical-shaped extremity support of the reference noodle (7) associated with the cover and the main plate (26).

8. Device according to one of the preceding claims, **characterised in that** the alveoles are embodied via the thermoforming of a main thermoplastic plate (26) welded to an associated plate (29) so as to form the bottom of the alveoles (11, 12), whereas each module (30) comprises a sealing device (34) supporting stoppers (15) and thermoformed extremity supports (20, 21), said module being cut from the main plate (26) along a tracing (31) comprising three sides and the fourth being articulated by means of precut lines (32, 33).

9. Device according to claim 8, **characterised in that** the main plate (26) is transparent, the face forming the bottom of the alveoles (11, 12) of the associated plate (29) being slightly uneven, the stoppers (15) penetrating sufficiently into their respective alveole (11, 12) so as to crush the blood drop and make one portion flow back to a swollen portion (44) situated at the base of the alveoles (11, 12).

10. Device according to claim 1, **characterised in that** the alveoles are formed from independent containers (47) clipped onto a fixing support (50).

11. Device according to claims 1 and 8, **characterised in that** one alveole (51) is associated with a housing (54) communicating with said alveole by at least one channel (56), the alveoles and channels being thermoformed in a main thermoplastic plate (52) and closed by an associated plate (53), the housing (54) containing a reactive liquid container (55) able to be crushed so as to free the reactive liquid it contains and direct it towards the alveole (51) by means of the channel (56).

12. Device according to claims 1 and 8, **characterised in that** it comprises between two and four modules distributed along two sides (39) which fold onto each other along a median hinge zone (40) so as to form a pocket whose edges are formed of protuberances (41,42) thermoformed from the main plate (26) and which are nested so as to support the closed pocket.

13. Device according to claim 8, **characterised in that** the plates (26, 29) are made of rigid PVC and welded by high frequency weld.

## Patentansprüche

1. Testvorrichtung einer Flüssigkeit durch Vermischen jeder von ihnen mit einer oder mehreren flüssigen Reagenzien, umfassend wenigstens zwei Module (1, 2) mit zwei Zellen (3, 4), die jede eine unterschiedliche flüssige Reagenzie derart enthalten, dass jede Zelle jedes Moduls jeweils einer Zelle jedes der anderen Module entspricht, die dieselbe flüssige Reagenzie enthalten, wobei die Zellen (3, 4) nach dem Test durch eine Stöpsel (17) enthaltende manuelle Verschlussvorrichtung (25) geschlossen werden, die mit den Zellen (3, 4) durch ein mechanisches Mittel verbunden ist, **dadurch gekennzeichnet, dass** die Verschlussvorrichtung (25) dieselbe Anzahl Stöpsel (17) enthält wie das Modul Zellen umfasst, wobei besagte Verschlussvorrichtung jedem Modul durch eine elastische mechanische Verbindung (18) zugeordnet ist, die eine Faltzone bildet, die das Zusammenfalten der Verschlussvorrichtung (25) auf den Modulen (1, 2) erlaubt und damit den Austausch der Stöpsel verhindert.

2. Vorrichtung gemäß Anspruch 1, **dadurch gekennzeichnet, dass** der Verschluss der Zellen vor dem Test durch eine Folie (16) gewährleistet wird, die auf dem Rand der Öffnung (9) der Zellen (3, 4) aufgeschweißt und geeignet ist, durch Abziehen abgenommen zu werden.

3. Vorrichtung gemäß Anspruch 1, **dadurch gekennzeichnet, dass** der Verschluss der Zellen (11, 12) nach der Einführung der flüssigen Reagenzien durch dieselbe manuelle Verschlussvorrichtung (14) zum Wiederverschließen der Zellen (11, 12), insbesondere durch Stöpsel (15) gewährleistet wird.

4. Vorrichtung gemäß Anspruch 1 bis 3, **dadurch gekennzeichnet, dass** eine der Zellen (3, 4) jedes Moduls Testserum Anti-A und das andere Testserum Anti-B enthält, wobei das erste Modul (1) dazu bestimmt ist, das Empfängerblut aufzunehmen und jedes andere Modul (2) dazu bestimmt ist, das Blut eines Spenders aufzunehmen, das aus einem gebogenen Probestab (7) extrahiert worden ist.

5. Vorrichtung gemäß Anspruch 4, **dadurch gekennzeichnet, dass** eine wiederverschließbare Lageraushöhlung (6) des gebogenen Probestabes (7) jedem Modul (2) zugeordnet ist.

6. Vorrichtung gemäß Anspruch 4, **dadurch gekennzeichnet, dass** der gebogene Probestab (7) nach Gebrauch an seinen Enden (22, 23) , die während der Durchführung des Tests abgeschnitten wurden, an Endstützen (20, 21, 24) befestigt wird, die entweder in einer Lageraushöhlung (5) oder auf der Verschlussvorrichtung (46) befestigt werden, die als Deckel der Lageraushöhlung (6) dient.

7. Vorrichtung gemäß Anspruch 6, **dadurch gekennzeichnet, dass** die Verschlussvorrichtung (35) in geschlossener Position der Zellen nach dem Test den gebogenen Probestab (7) zwischen der Endstütze in zylindrischer und /- oder konischer Form des Probestabes (7) festklemmt, der dem Deckel und der Hauptplatte (26) zugeordnet ist.

8. Vorrichtung gemäß einem der vorgenannten Ansprüche, **dadurch gekennzeichnet, dass** die Zellen durch Thermoformung einer thermoplastischen Hauptplatte (26) realisiert sind, die an eine zugeordnete Platte (29) geschweißt ist, um den Boden der Zellen (11, 12) zu bilden, während jedes Modul (30) eine Verschlussvorrichtung (34) umfasst, die Stöpsel (15) und thermogeformte Endstützen (20, 21) stützen, die aus der Hauptplatte (26) gemäß einer Markierung (31) ausgeschnitten sind, die drei Seiten umfasst, wobei die vierte mittels vorausgeschnittener Linien (32, 33) artikuliert ist.

9. Vorrichtung gemäß Anspruch 8, **dadurch gekennzeichnet, dass** die Hauptplatte (26) transparent ist, wobei die den Boden der Zellen (11, 12) bildenden Seite der zugeordneten Platte (29) leicht rau ist, die Stöpsel (15) dringen ausreichend in das Innere ihrer jeweiligen Zelle (11, 12) ein, um dort den Blutstropfen zu zerdrücken und dabei einen Teil zu einer Verstärkung (44) zurückzudrängen, der sich an der Basis der Zellen (11, 12) befindet.

10. Vorrichtung gemäß Anspruch 1, **dadurch gekennzeichnet, dass** die Zellen aus unabhängigen Behältern (47) gebildet sind, die auf einer Befestigungsstütze (50) aufgeklipst sind.

11. Vorrichtung gemäß Anspruch 1 und 8, **dadurch gekennzeichnet, dass** eine Zelle (51) einer Aufnahme (54) zugeordnet ist, die mit besagter Zelle durch wenigstens einen Kanal (56) in Verbindung steht, wobei Zellen und Kanäle in einer thermoplastischen Hauptplatte (52) thermogeformt und durch eine zugeordnete Platte (53) geschlossen sind, wobei die Aufnahme (54) einen Behälter einer flüssiger Reagenzie (55) enthält, der geeignet ist, zerdrückt zu werden, um die flüssige Reagenzie freizusetzen, die er enthält, und sie durch den Kanal (56) zur Zelle (51) zu leiten.

12. Vorrichtung gemäß Anspruch 1 und 8, **dadurch gekennzeichnet, dass** sie zwei bis vier Module umfasst, die gemäß zwei Seiten (39) verteilt sind, die sich gemäß einer zentralen Scharnierzone (40) um sich selbst knicken, um eine Tasche zu bilden, deren Ränder aus aus der Hauptplatte (26) thermogeformten Protuberanzen (41, 42) gebildet werden und die in einander eingreifen, um die geschlossene Tasche zu bilden.

13. Vorrichtung gemäß Anspruch 8, **dadurch gekennzeichnet, dass** die Platten (26, 29) aus steifem und durch Hochfrequenzschweißen verschweißtem Polyvinylchlorid sind.
